# FASCICULE DE BREVET EUROPEEN

(11) **EP 1 681 047 B1**
(45) Date de publication et mention de la délivrance du brevet: **28.11.2012**
(21) Numéro de dépôt: 06290076.6
(22) Date de dépôt: 12.01.2006
(51) Int. Cl.: A61K 8/81, A61K 8/87, A61K 8/04, A61K 8/22, A61K 8/34, A61Q 5/06

(54) **Composition cosmétique comprenant au moins un polyuréthane fixant non associatif et au moins un polymère sulfoné**
Zusammensetzung enthaltend mindestens ein fixierendes nicht.assoziatives Polyurethan und mindestens ein Sulfonpolymer
Cosmetic composition comprising at least one fixing non-associative polyurethane and at least one sulfonated polymer

(30) Priorité: 13.01.2005 FR 0500367
(43) Date de publication de la demande: 19.07.2006
(73) Titulaire: L'Oréal, 75008 Paris (FR)
(72) Inventeur: Pataut, Françoise, 75017 Paris (FR); Gringore, Charles, 92600 Asnières-sur-Seine (FR)
(74) Mandataire: Casalonga, Axel

(56) Documents cités:
- EP-A- 1 306 078
- EP-A- 1 488 778
- EP-A- 1 518 538
- FR-A- 2 783 166
- FR-A- 2 785 182
- FR-A- 2 819 177
- US-B1- 6 383 472

## Description

La présente invention est relative à une composition de traitement cosmétique capillaire comprenant un polyuréthane fixant non associatif et un polymère sulfoné particulier, ainsi qu'à un procédé de traitement cosmétique mettant en oeuvre cette composition.

Les produits coiffants, tels que des laques, des mousses et des gels, contiennent généralement des polymères fixants anioniques ou non ioniques et en particulier des polymères de type polyuréthanes. Ces produits sont habituellement utilisés pour structurer la coiffure et/ou lui apporter une tenue durable.

Cependant l'étalement sur la chevelure n'est pas aisé car la fixation de la coiffure est quasi-immédiate et il est difficile d'étaler le produit en particulier avec les mains sur l'ensemble de la chevelure.

On connaît de la demande de brevet FR 2783166 une composition cosmétique capillaire comprenant, dans un milieu cosmétiquement acceptable, au moins une silicone arylée et au moins un copolymère diméthiconol/isophorone.

De même, la demande de brevet FR 2785182 décrit un dispositif flacon pompe muni d'un réservoir contenant une composition capillaire comprenant, dans un milieu cosmétiquement acceptable, au moins un polycondensat (A) ayant une séquence polyuréthane et/ou polyurée et au moins un polymère filmogène (B) différent de (A).

Par ailleurs, la demande de brevet EP 1488778 concerne une composition cosmétique capillaire comprenant, dans un milieu cosmétiquement acceptable, de l'isocicosane et au moins un polyuréthane fixant siliconé.

Il est également du document FR 2819177 une composition pour application topique, comprenant au moins un agent conditionneur soluble particulier et au moins un polymère amphiphile utilisée notamment pour le traitement et le soin des cheveux.

Enfin, il est connu de la demande de brevet EP 1306078 une composition de traitement cosmétique des cheveux contenant en association au moins un polyuréthane fixant non associatif et au moins un polyuréthane associatif anionique ou non ionique, et conditionnée dans un dispositif aérosol.

La demanderesse a trouvé de manière surprenante qu'en associant un polyuréthane fixant non associatif particulier et un polymère associatif particulier comprenant un motif dérivé d'un monomère à insaturation éthylénique à groupement sulfonique non aromatique, on obtenait une mise en forme de la coiffure facilitée, en particulier par un simple travail aux doigts sans obtenir le durcissement et le figeage instantanés classiquement rencontrés avec les produits habituels de fixation.

Cette composition présente en outre l'avantage de bien s'étaler sur les cheveux et l'application peut se faire aussi bien sur les cheveux secs que sur cheveux humides.

Dans le cas d'une application sur cheveux humides, un séchage à l'air libre ou au sèche-cheveux peut être réalisé. Le résultat est une coiffure souple et naturelle.

L'invention a donc pour objet une composition cosmétique de traitement capillaire telle que décrite ci-dessous.

Un autre objet de la présente invention est un procédé de traitement cosmétique des cheveux mettant en oeuvre la composition selon l'invention.

L'invention a encore pour objet l'utilisation de la composition selon l'invention comme produit de coiffage.

D'autres objets, caractéristiques, aspects et avantages de l'invention apparaîtront encore plus clairement à la lecture de la description et des divers exemples qui suivent.

La présente invention concerne une composition de traitement cosmétique capillaire comprenant, dans un milieu cosmétiquement acceptable, au moins un polyuréthane fixant non associatif ledit polyuréthane étant un polycondensat comprenant au moins une séquence polyuréthane susceptible d'apporter du maintien à la coiffure et ne comprenant pas dans sa structure de chaîne grasse terminale ou pendante comportant plus de 10 atomes de carbone et au moins un polymère sulfoné associatif partiellement ou totalement neutralisé ou non, comprenant au moins un motif dérivé d'un monomère à insaturation éthylénique à groupement sulfonique non aromatique ledit polymère sulfoné associatif possédant au moins une partie hydrophile et au moins une chaîne grasse terminale ou pendante comportant au moins 10 atomes de carbone.

Par monomère à insaturation éthylénique à groupement sulfonique non aromatique, on entend un monomère possédant au moins une double liaison et porteur dans sa structure d'un groupement SO₃X (avec X désignant un hydrogène ou des ions d'un métal alcalin, ou alcalino terreux ou des ions ammonium ou encore des ions issus d'une amine organique) non directement fixé sur un noyau aromatique.

Par milieu cosmétiquement acceptable, on entend un milieu compatible avec les cheveux, mais aussi d'odeur, d'aspect et de toucher agréables.

Par polymère fixant, on entend tout polymère permettant de conférer une forme ou de maintenir une forme sur une chevelure donnée.

Par polyuréthane fixant non associatif, on entend au sens de la présente invention, des polycondensats fixants comprenant au moins une séquence polyuréthane susceptible d'apporter du maintien à la coiffure et qui ne comprennent pas dans leur structure de chaîne grasse terminale ou pendante comportant plus de 10 atomes de carbone. Ils sont décrits en particulier dans les brevets EP 0 751 162, EP 0 637 600, FR 2 743 297 et EP 0 648 485, ainsi que les brevets EP 0 656 021, WO 94/03510 et EP 0 619 111.

Les polyuréthanes fixants non associatifs utilisés conformément à l'invention peuvent être solubles dans le milieu cosmétiquement acceptable, notamment après neutralisation par une base organique ou minérale, ou encore former une dispersion dans ce milieu. La dispersion peut comprendre alors au moins 0,05 % de tensioactif permettant la mise en dispersion et le maintien en dispersion du polyuréthane fixant non associatif.

Selon l'invention, on peut utiliser tout type de tensioactif dans ladite dispersion, mais de préférence un tensioactif non ionique. La taille moyenne des particules du polyuréthane fixant non associatif dans la dispersion est de préférence comprise entre 0,1 et 1 micron.

De préférence, les polyuréthanes fixants non associatifs utilisés sont solubles dans le milieu.

A titre d'exemple, le polyuréthane fixant non associatif peut être formé par un arrangement de blocs, cet arrangement étant obtenu notamment à partir de :
(1) au moins un composé qui contient deux ou plus de deux atomes d'hydrogène actifs par molécule ;
(2) au moins un diol ou un mélange de diols contenant des fonctions acides ou leurs sels ; et
(3) au moins un di- ou polyisocyanate.

Avantageusement, les composés (1) sont choisis dans le groupe comprenant les diols, les diamines, les polyesterols, les polyétherols ou leurs mélanges.

Les composés (1) comprenant deux ou plus deux atomes d'hydrogène actifs par molécule qui sont préférés, sont les polyéthylèneglycols et les polypropylèneglycols linéaires, en particulier ceux qui sont obtenus par réaction de l'oxyde d'éthylène ou de propylène avec l'eau ou du diéthylène ou du dipropylèneglycol en présence d'hydroxyde de sodium en tant que catalyseur. Ces polyalkylèneglycols ont généralement une masse moléculaire comprise entre environ 600 et 20 000.

D'autres composés organiques préférés sont ceux qui ont des groupes mercapto, amino, carboxyle ou hydroxyle. Parmi ceux-ci, on cite plus particulièrement les composés polyhydroxylés tels que les polyéther-diols, les polyester-diols, les polyacétal-diols, les polyamide-diols, les polyester-polyamide-diols, les poly(alkylène éther)-diols, les polythioéther-diols et les polycarbonate-diols.

Les polyéther-diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats d'oxyde d'alkylène. Des polyéthers appropriés sont par exemple préparés par condensation d'oxydes d'alkylène et d'alcools polyhydriques, tels que l'éthylèneglycol, le 1,2-propylèneglycol et le 1,4-butanediol.

Les polyester-diols, polyester-amides, polyamide-diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylèneglycol, le 1,2-propylèneglycol, le 1,4-butanediol, le néopentylglycol et l'hexanediol. On peut aussi utiliser des aminoalcools, par exemple l'éthanolamine. Des diamines appropriées pour préparer les amide-polyesters sont l'éthylène-diamine et l'hexaméthylène-diamine.

Des polyacétals appropriés peuvent être préparés, par exemple, à partir de 1,4-butanediol ou d'hexanediol et de formaldéhyde. Des polythioéthers appropriés peuvent être préparés par exemple par réaction de condensation entre des thioglycols seuls ou en combinaison avec d'autres glycols tels que l'éthylèneglycol, le 1,2-propylèneglycol ou avec d'autres composés polyhydroxylés. Les composés polyhydroxylés contenant déjà des groupements uréthannes, des polyols naturels, qui peuvent être davantage modifiés, par exemple, l'huile de castor et les carbohydrates peuvent également être utilisés.

Plus préférentiellement, le composé du groupe (1) est un polyestérol, notamment un polyester-diol formé par la réaction d'au moins un (di)-polyol (1ₐ) et d'au moins un acide (1_{b}). Le (di)- polyol (1ₐ) est en particulier choisi dans le groupe comprenant le néopentylglycol, le 1,4-butanediol, l'hexanediol, l'éthylèneglycol, le diéthylèneglycol, le propylèneglycol, le butylèneglycol, le néopentylglycol et (di)-polyéthylèneglycol. L'acide (1_{b}) est en particulier choisi dans le groupe comprenant l'acide phtalique, l'acide isophtalique, l'acide adipique et l'acide (poly)-lactique.

En tant que composé (2), diol ou un mélange de diol contenant des fonctions acides ou leurs sels, on peut notamment utiliser un acide hydroxycarboxylique tel que l'acide diméthylol-propanoïque (DMPA) ou un acide 2,2-hydroxyméthyl-carboxylique. En général, le composé (2) est utile en tant que bloc de couplage. En tant que composés (2), on préfère ceux comprenant au moins un poly (acide-(alpha-hydroxycarboxyliquediol).

Les composés (2) particulièrement préférés conformément à l'invention sont ceux choisis dans le groupe comprenant l'acide 2,2-di-(hydroxyméthyl)acétique, l'acide 2,2-dihydroxyméthyl-propionique, l'acide 2,2-dihydroxyméthylbutyrique, l'acide 2,2-dihydroxyméthylpentanoïque.

Le di- ou polyisocyanate (3) peut être choisi en particulier dans le groupe comprenant l'hexaméthylène-diisocyanate, l'isophorone-diisocyanate (IDPI), le toluylène-diisocyanate, le diphénylméthane-4,4'-diisocyanate (DPMD) et le dicyclohexyl-méthane-4,4'-diisocyanate (DCMD), le méthylène-di-p-phényl-diisocyanate, le méthylène-bis(4-cyclohexylisocyanate), les toluène-diisocyanates, le 1,5-naphtalène-diisocyanate, le 4,4'-diphényl-méthane-diisocyanate, le 2,2'-diméthyl-4,4'-diphénylméthane-diisocyanate, le 1,3-phénylène-diisocyanate, le 1,4-phénylène diisocyanate, des mélanges de 2,4- et de 2,6-toluène-diisocyanates, le 2,2'-dichloro-4,4'-diisocyanato-diphénylméthane, le 2,4-dibromo-1,5-diisocyanato naphtalène, le butane-1,4-diisocyanate, l'hexane-1,6-diisocyanate, le cyclohexane-1,4-diisocyanate.

Le polyuréthane fixant non associatif peut être formé à l'aide d'un composé supplémentaire (4) servant en général à allonger sa chaîne. Ces composés (4) peuvent être choisis dans le groupe comprenant notamment les glycols saturés ou insaturés tels que l'éthylèneglycol, le diéthylèneglycol, le néopentylglycol, le triéthylène glycol ; les aminoalcools tels que l'éthanolamine, la propanolamine, la butanolamine ; les amines primaires hétérocycliques, aromatiques, cycloaliphatiques, et aliphatiques ; les diamines ; les acides carboxyliques tels que les acides carboxyliques aliphatiques, aromatiques et hétérocycliques comme les acides oxalique, succinique, glutarique, adipique, sébacique et téréphtalique; les acides aminocarboxyliques. Les composés (4) préférés sont les diols aliphatiques.

Les polyuréthanes fixants non associatifs utilisés selon l'invention peuvent également être formés à partir de composés supplémentaires (5) ayant un squelette siliconé tels que les polysiloxanes, les polyalkylsiloxanes ou les polyarylsiloxanes, notamment les polyéthylsiloxanes, les polyméthylsiloxanes et les polyphénylsiloxanes, comportant éventuellement des chaînes hydrocarbonées greffées sur les atomes de silicium.

Les polyuréthanes fixants non associatifs utilisés avantageusement comprennent un motif répétitif de base répondant à la formule générale (I) :

-O-B-O-CO-NH-R-NH-CO- (I)

dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes alkylènes de type aromatique, aliphatique en C₁ à C₂₀, cycloaliphatique en C₁ à C₂₀, ces groupes étant substitués ou non.

Le groupe R est avantageusement choisi parmi les groupes répondant aux formules suivantes : 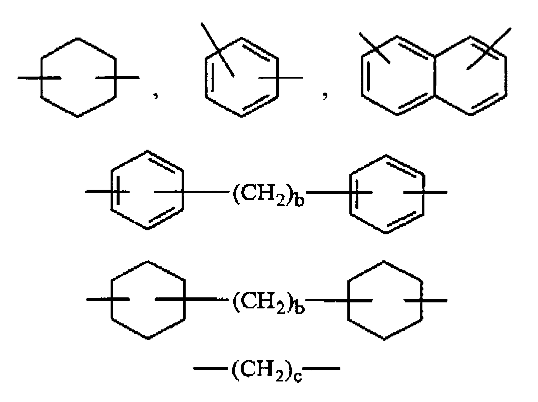 dans lesquelles b est un nombre entier compris entre 0 et 3, et c un nombre entier compris entre 1 et 20, de préférence compris entre 2 et 12.

En particulier, le groupe R est choisi parmi les groupes hexaméthylène, 4,4'-biphénylèneméthane, 2,4- et/ou 2,6-tolylène, 1,5-naphtylène, p-phénylène, méthylène- 4,4-bis-cyclohexyle et le groupe divalent dérivé de l'isophorone.

Le polyuréthane fixant non associatif utilisé dans la présente invention peut avantageusement comprendre en outre au moins une séquence polysiloxane dont le motif répétitif de base répond par exemple à la formule générale (II) :

-O-P-O-CO-NH-R-NH-CO- (II)

dans laquelle :
- P est un segment polysiloxanique, et
- R est un groupe divalent choisi parmi les groupes alkylènes de type aromatique, aliphatique en C₁ à C₂₀, cycloaliphatique en C₁ à C₂₀, ces groupes étant substitués ou non.

Avantageusement, le segment polysiloxanique P répond à la formule générale (III) ci-après : 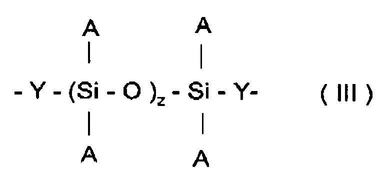 dans laquelle:
- les groupes A, qui peuvent être identiques ou différents, sont choisis parmi d'une part les groupes hydrocarbonés monovalents en C₁ à C₂₀ exempts ou substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne du segment polysiloxane soit comprise entre 300 et 10 000.

En général, le groupe divalent Y est choisi parmi les groupes alkylènes de formule -(CH₂)ₐ- ,dans laquelle a représente un nombre entier pouvant être compris entre 1 et 10.

Les groupes A peuvent être choisis parmi les groupes alkyles, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle ; les groupes arylalkyle, notamment benzyle et phényléthyle, ainsi que les groupes tolyle et xylyle.

A titre d'exemples de polyuréthane fixant non associatif, on peut notamment citer le copolymère acide diméthylolpropionique/ isophorone-diisocyanate/néopentylglycol/polyesterdiols (connu aussi sous le nom de polyuréthane-1, appellation INCI) vendu sous la marque Luviset® PUR par la société BASF, et le copolymère acide diméthylolpropionique/isoprène-diisocyanate/néopentylglycol/polyesterdiols/diamine siliconée (connu aussi sous le nom de polyuréthane-6, appellation INCI) vendu sous la marque Luviset® Si PUR A par la société BASF.

Les polyuréthanes fixants non associatifs sont de préférence utilisés en une quantité de 0,1 % à 30 % en poids, plus préférentiellement entre 0,2 % et 15 % en poids, et encore plus préférentiellement de 0,5 % à 10 % en poids par rapport au poids total de la composition de traitement des cheveux.

Les polymères sulfonés conformes à l'invention sont des polymères associatifs sulfonés non neutralisés ou partiellement ou totalement neutralisés, comprenant au moins un motif dérivé d'un monomère à insaturation éthylénique à groupement sulfonique non aromatique et comprenant, au moins une partie hydrophobe.

Par polymère associatif, on entend au sens de la présente invention, tout polymère possédant au moins une partie hydrophile et au moins une chaîne grasse terminale ou pendante comportant au moins 10 atomes de carbone. Ce type de polymère est susceptible d'interagir avec lui-même ou avec des composés particuliers tels que les tensioactifs pour conduire à un épaississement du milieu.

Par partiellement neutralisé, on entend au sens de la présente invention des groupes sulfoniques sous forme salifiée présents dans le polymère à raison de 1 % à 99 %.

La partie hydrophobe, présente dans les polymères de l'invention comporte de préférence de de 10 à 18 atomes de carbone et encore plus particulièrement de 12 à 18 atomes de carbone.

De façon préférentielle, les polymères sulfonés conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères sulfonés conformes à l'invention ont généralement un poids moléculaire moyen en nombre allant de 1000 à 20 000 000 g/mole, de préférence allant de 20 000 à 5 000 000 g/mole et plus préférentiellement encore de 100 000 à 1 500 000 g/mole.

Les polymères sulfonés selon l'invention peuvent être réticulés ou non-réticulés.

De préférence, on choisit des polymères sulfonés réticulés.

Lorsqu'ils sont réticulés, les agents de réticulation peuvent être choisis parmi les composés à polyinsaturation oléfinique couramment utilisés pour la réticulation des polymères obtenus par polymérisation radicalaire.

On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

Les monomères à insaturation éthylénique à groupement sulfonique non aromatique sont choisis notamment parmi l'acide vinylsulfonique, les acides (méth)acrylamido(C₁₋C₂₂)alkylsulfoniques, les acides N-(C₁-C₂₂)alkyl(méth)acrylamido(C₁₋C₂₂)alkylsulfoniques comme l'acide undécyl-acrylamido-méthane-sulfonique ainsi que leurs formes partiellement ou totalement neutralisées.

Plus préférentiellement, on utilisera les acides (méth)acrylamido(C₁-C₂₂)alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

Plus particulièrement, on utilisera l'acide 2-acrylamido-2-méthylpropane-sulfonique (AMPS) ainsi que ses formes partiellement ou totalement neutralisées.

Les polymères sulfonés conformes à l'invention peuvent notamment être choisis parmi les polymères amphiphiles statistiques d'AMPS modifiés par réaction avec une n-monoalkylamine ou une din-alkylamine en C₆-C₂₂, et tels que ceux décrits dans la demande de brevet WO00/31154 (faisant partie intégrante du contenu de la description). Ces polymères peuvent également contenir en outre d'autres motifs dérivés de monomères à insaturation éthylénique ne comportant pas de chaîne grasse.

Ces monomères à insaturation éthylénique ne comportant pas de chaîne grasse peuvent être choisis par exemple parmi les acides (méth)acryliques, leurs dérivés alkyl substitués en β ou leurs esters obtenus avec des monoalcools ou des mono- ou polyalkylèneglycols, les (méth)acrylamides, la vinylpyrrolidone, l'anhydride maléique, l'acide itaconique ou l'acide maléique ou les mélanges de ces composés.

Les polymères sulfonés peuvent être choisis parmi les copolymères amphiphiles d'AMPS et d'au moins un monomère hydrophobe à insaturation éthylénique comportant au moins une partie hydrophobe ayant de 10 à 18 atomes de carbone et encore plus particulièrement de 12 à 18 atomes de carbone.

Ces mêmes copolymères peuvent contenir en outre un ou plusieurs motifs dérivés de monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :
« Self-assembling amphiphilic polyelectrolytes and their nanostructures - Chinese Journal of Polymer Science Vol. 18, N°40, (2000), 323-336. »
« Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
« Solution properties of miscelle networks formed by non-ionic moieties covalently bound to an polyelectrolyte : salt effects on rheological behavior - Langmuir, 2000, Vol.16, N°12, 5324-5332 » ;
« Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Les monomères hydrophobes à insaturation éthylénique de ces copolymères particuliers sont choisis de préférence parmi les acrylates ou les acrylamides de formule (I) suivante : 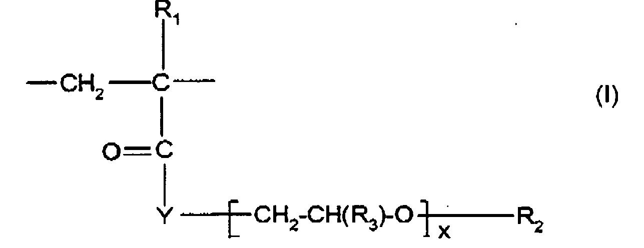 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant au moins de 10 à 18 atomes de carbone et encore plus particulièrement de 12 à 18 atomes de carbone ; x désigne un nombre de moles d'oxyde d'alkylène et varie de 0 à 100.

Le radical R₂ est choisi de préférence parmi les radicaux alkyles n-décyle, n-hexadécyle, n-dodécyle ramifiés ou cycliques (par exemple cyclododécane (C₁₂) ou adamantane (C₁₀)) ; les radicaux alkylperfluorés de formule - (CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée de l'invention, le monomère de formule (I) comporte au moins un motif oxyde d'alkylène (x ≥ 1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Parmi ces polymères sulfonés, on peut citer :
les copolymères d'AMPS partiellement ou totalement neutralisé et de méthacrylate de dodécyle ainsi que les copolymères réticulés ou non-réticulés d'AMPS partiellement ou totalement neutralisé et de n-dodécylméthacrylamide, tels que ceux décrits dans les articles de Morishima cités ci-dessus.
   On citera plus particulièrement les copolymères constitués de motifs acide 2-acrylamido-2-méthylpropane-sulfonique (AMPS) de formule (II) suivante : 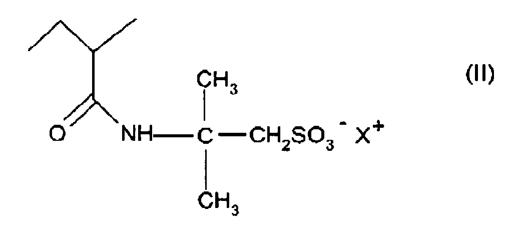
dans laquelle X⁺ est un proton, un cation de métal alcalin, un cation alcalino-terreux ou l'ion ammonium,
et de motifs de formule (III) suivante : 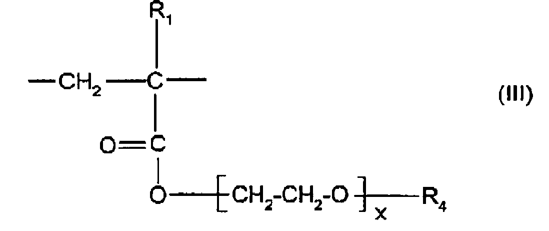 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₁₀-C₂₂.

Les polymères sulfonés particulièrement préférés sont ceux pour lesquels x = 25 , R₁ désigne un méthyle et R₄ représente le n-dodécyle; ils sont décrits dans les articles de Morishima mentionnés ci-dessus.

Les polymères pour lesquels X⁺ désigne le sodium ou l'ammonium sont plus particulièrement préférés.

Les polymères sulfonés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthyl-valéronitrile, le ABAH (2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.

En utilisant la polymérisation par précipitation dans le tert-butanol, il est possible d'obtenir une distribution de la taille des particules du polymère particulièrement favorable pour ses utilisations.

La distribution de la taille des particules du polymère peut être déterminée par exemple par diffraction laser ou analyse d'image.

Une distribution intéressante pour de type de polymère et déterminée par analyse d'image est la suivante : 60,2% inférieur à 423 microns, 52,0% inférieur à 212 microns, 26,6% inférieur à 106 microns, 2,6% inférieur à 45 microns et 26,6% supérieur à 850 microns.

La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et,
- d'un alcool en C₁₀-C₁₈ oxyéthyléné par 8 moles d'oxyde d'éthylène (GENAPOL® C-080 de la société HOECHST/CLARIANT),
- d'un alcool oxo en C₁₁ oxyéthyléné par 8 moles d'oxyde d'éthylène (GENAPOL® UD-080 de la société HOECHST/CLARIANT),
- d'un alcool oxo en C₁₁ oxyéthyléné par 7 moles d'oxyde d'éthylène (GENAPOL® UD-070 de la société HOECHST/CLARIANT),
- d'un alcool en C₁₂-C₁₄ oxyéthyléné par 7 moles d'oxyde d'éthylène (GENAPOL® LA-070 de la société HOECHST/CLARIANT),
- d'un alcool en C₁₂-C₁₄ oxyéthyléné par 9 moles d'oxyde d'éthylène (GENAPOL® LA-090 de la société HOECHST/CLARIANT),
- d'un alcool en C₁₂-C₁₄ oxyéthyléné par 11 moles d'oxyde d'éthylène (GENAPOL® LA-110 de la société HOECHST/CLARIANT),
- d'un alcool en C₁₆-C₁₈ oxyéthyléné par 8 moles d'oxyde d'éthylène (GENAPOL® T-080 de la société HOECHST/CLARIANT),
- d'un alcool en C₁₆-C₁₈ oxyéthyléné par 15 moles d'oxyde d'éthylène (GENAPOL® T-150 de la société HOECHST/CLARIANT),
- d'un alcool en C₁₆-C₁₈ oxyéthyléné par 11 moles d'oxyde d'éthylène (GENAPOL® T-110 de la société HOECHST/CLARIANT),
- d'un alcool en C₁₆-C₁₈ oxyéthyléné par 20 moles d'oxyde d'éthylène (GENAPOL® T-200 de la société HOECHST/CLARIANT),
- d'un alcool en C₁₆-C₁₈ oxyéthyléné par 25 moles d'oxyde d'éthylène (GENAPOL® T-250 de la société HOECHST/CLARIANT),
- d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

La concentration molaire en % des motifs de formule (II) et des motifs de formule (III) dans les polymères selon l'invention variera en fonction de l'application cosmétique souhaitée et des propriétés rhéologiques de la formulation recherchées. Elle peut varier entre 0,1 et 99,9% en moles.

De préférence pour les polymères les plus hydrophobes, la proportion molaire en motifs de formule (I) ou (III) varie de 50, 1 à 99,9%, plus particulièrement de 70 à 95% et encore plus particulièrement de 80 à 90%.

De préférence pour les polymères peu hydrophobes, la proportion molaire en motifs de formule (I) ou (III) varie de 0,1 à 50%, plus particulièrement de 5 à 25% et encore plus particulièrement de 10 à 20%.

La distribution des monomères dans les polymères de l'invention peut être, par exemple, alternée, bloc (y compris multibloc) ou quelconque.

Selon l'invention, on préfère que les polymères aient des chaînes pendantes sensibles à la chaleur et dont la solution aqueuse présente une viscosité qui, au-delà d'une certaine température seuil, augmente, ou demeure pratiquement constante quand la température augmente.

Plus particulièrement encore, on préfère les polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cette optique, on préfère que la viscosité des solutions de polymère en dessous de la première température seuil soit de 5 à 50%, en particulier de 10 à 30% de la viscosité maximum à la seconde température seuil.

Ces polymères, de préférence, conduisent dans l'eau à un phénomène de démixion par chauffage se traduisant par des courbes présentant, en fonction de la température et de la concentration, un minimum appelé LCST (Lower Critical Solution Temperature).

Les viscosités (mesurées à 25°C au viscosimètre Brookfield aiguille 7) des solutions aqueuses à 1% vont de préférence de 20 000 mPa.s à 100 000 mPa.s et plus particulièrement de 60 000 mPa.s à 70 000 mPa.s.

Les polymères sulfonés conformes à l'invention sont de préférence présents en une quantité allant de 0,01 à 20 % en poids, mieux encore de 0,1 à 10 % en poids et encore plus préférentiellement de 0,2 % à 5 % en poids par rapport au poids total de la composition de traitement de cheveux.

De préférence, le rapport pondéral polyuréthane(s) fixant(s) non associatif(s)/polymère(s) sulfoné(s) est supérieur à 1,30.

Le milieu cosmétiquement acceptable comprend de l'eau et/ou un solvant cosmétiquement acceptable tel qu'un alcool inférieur en C₁-C₄, tel que l'éthanol, l'isopropanol, le tertio-butanol, le n-butanol ; les alyènepolyols comme le propylène glycol ; les éthers de polyols ; et leurs mélanges.

La composition selon l'invention peut comprendre en outre des additifs cosmétiques usuels choisis parmi les agents adhésifs, les agents réducteurs comme les thiols, les corps gras, les agents épaississants autres que les polymères sulfonés de l'invention, les agents nacrants, les adoucissants, les agents anti-mousse, les filtres solaires, les agents antiperspirants, les agents acidifiants, les agents alcalinisants, les colorants, les pigments, les parfums, les conservateurs, les tensioactifs, les silicones volatiles ou non, les huiles végétales, minérales, ou de synthèse, les protéines et les vitamines et leurs mélanges.

L'homme du métier veillera à choisir les éventuels additifs et leur quantité de manière à ce qu'ils nuisent pas aux propriétés des compositions de l'invention.

Ces additifs sont présents dans la composition selon l'invention en une quantité allant de 0 à 10 % en poids par rapport au poids total de la composition.

Les compositions conformes à l'invention peuvent se présenter sous forme de gel, d'un liquide ou d'une mousse et être utilisées en application rincée ou non. Elles peuvent être conditionnées dans un tube, vaporisateur ou dans un dispositif aérosol usuel en cosmétique.

Les agents propulseurs utilisés dans les systèmes aérosols selon l'invention peuvent être des gaz liquéfiés ou comprimés et sont en particulier choisis parmi les alcanes en C₃C₅, le DME, les fluoroalcanes tels que le Dymel 152A ou leurs mélanges.

Les compositions conformes à l'invention peuvent être utilisées en tant que compositions de fixation et/ou de maintien des cheveux, compositions de soin de cheveux, shampoings, compositions de conditionnement des cheveux, telles que des compositions destinées à apporter de la douceur aux cheveux, ou encore des compositions de maquillage de cheveux.

La présente invention concerne également un procédé de traitement cosmétique des cheveux qui consiste à appliquer une quantité efficace d'une composition telle que décrite ci-dessus, sur les cheveux, à rincer ou non après un éventuel temps de pose.

Selon un mode de réalisation préféré de l'invention, la composition peut être utilisée comme produit de coiffage non rincé.

L'exemple suivant illustre la présente invention.

### Exemple :

On prépare un produit de coiffage sous forme de spray aérosol. Les quantités sont indiquées en % en poids :
- Luviset® Si PUR vendu par la société BASF 2%
- Acide polyacrylamidométhylpropane sulfonique neutralisé partiellement à l'ammoniaque réticulé proposé sous l'appellation Aristoflex HMS par la société CLARIANT 1%
- Ethanol 23%
- Diméthyléther 35%
- Eau qsp 100%

On pulvérise ce produit de coiffage sur la chevelure et on met en forme la coiffure. On obtient un bon maintien de la coiffure.

## Revendications

1. Composition de traitement cosmétique capillaire, **caractérisée en ce qu'**elle comprend, dans un milieu cosmétiquement acceptable, au moins un polyuréthane fixant non associatif ledit polyuréthane étant un polycondensat comprenant au moins une séquence polyuréthane susceptible d'apporter du maintien à la coiffure et ne comprenant pas dans sa structure de chaîne grasse terminale ou pendante comportant plus de 10 atomes de carbone et au moins un polymère sulfone associatif partiellement ou totalement neutralisé ou non, comprenant au moins un motif dérivé d'un monomère à insaturation éthylénique à groupement sulfonique non aromatique, ledit polymère sulfoné associatif possédant au moins une partie hydrophile et au moins une chaîne grasse terminale ou pendante comportant au moins 10 atomes de carbone.

2. Composition de traitement selon la revendication 1, **caractérisée en ce que** le polyuréthane fixant non associatif comprend un motif répétitif de base répondant à la formule générale (I) :
-O-B-O-CO-NH-R-NH-CO- (I)
dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes alkylènes de type aromatique, aliphatique en C₁ à C₂₀, cycloaliphatique en C₁ à C₂₀, ces groupes étant substitués ou non.

3. Composition de traitement selon la revendication 1, **caractérisée en ce que** le polyuréthane fixant non associatif comprend un motif répétitif de base répondant à la formule générale (II) :
-O-P-O-CO-NH-R-NH-CO- (II)
dans laquelle :
- P est un segment polysiloxanique, et
- R est un groupe divalent choisi parmi les groupes alkylènes de type aromatique, aliphatique en C₁ à C₂₀, cycloaliphatique en C₁ à C₂₀, ces groupes étant substitués ou non.

4. Composition de traitement selon la revendication 1 ou 2, **caractérisée en ce que** le polyuréthane fixant non associatif est un copolymère acide diméthylolpropionique/isophoronediisocyanate/néopentylglycol/ polyester diols.

5. Composition de traitement selon la revendication 1 ou 3, **caractérisée en ce que** le polyuréthane fixant non associatif est un copolymère acide diméthylolpropionique/isophoronediisocyanate /néopentylglycol/polyester diols/diamine siliconée.

6. Composition de traitement selon l'une quelconque des revendications précédentes, **caractérisée en ce que** le monomère à insaturation éthylénique à groupement sulfonique non aromatique est choisi parmi l'acide vinylsulfonique, les acides (méth)acrylamido (C₁-C₂₂)alkylsulfoniques, les acides N-(C₁-C₂₂)alkyl(méth)acrylamido(C₁-C₂₂)alkylsulfoniques ainsi que leurs formes partiellement ou totalement neutralisées.

7. Composition de traitement selon la revendication 6, **caractérisée en ce que** le monomère à insaturation éthylénique à groupement sulfonique non aromatique est choisi parmi l'acide acrylamido-méthane-sulfonique l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acryamido-n-butane-sulfonique, l'acide 2-acrylamido-2, 4, 4-triméthylpentane-sulfonique, l'acide 2-méthacrylamido-dodécyl-sulfonique, l'acide 2-acrylamide-2,6-diméthyl-3-heptane-sulfonique, ainsi que leurs formes partiellement ou totalement neutralisées.

8. Composition de traitement selon l'une quelconque des revendications 6 ou 7, **caractérisée en ce que** le monomère à insaturation éthylénique à groupement sulfonique non aromatique est l'acide 2-acrylamido-2-méthylpropane-sulfonique (AMPS), ainsi que ses formes partiellement ou totalement neutralisées.

9. Composition de traitement selon la revendication 8, **caractérisée en ce que** le ou les polymères sulfonés contiennent en outre au moins un motif dérivé d'un monomère à insaturation éthylénique ne comparant pas de chaîne grasse choisi parmi les acides (méth)acryliques et leurs dérivés alkyl substitués en ß, et leurs esters obtenus avec des mono alcools ou des mono- ou polyalkylèneglycols, ou bien parmi les (méth)acrylamides, la vinylpyrrolidone, l'anhydre maléique, l'acide itaconique ou l'acide maléique, ou les mélanges de ces composés

10. Composition de traitement selon l'une quelconque des revendications 1 à 8, **caractérisée en ce que** le ou les polymères sulfonés sont choisis parmi les copolymères amphiphiles d'AMPS et d'au moins un monomère hydrophobe à insaturation éthylénique comportant au moins une partie hydrophobe ayant de 10 à 18 atomes de carbone.

11. Composition de traitement selon la revendication 10, **caractérisée en ce que** la partie hydrophobe comporte de 12 à 18 atomes de carbone.

12. composition de traitement selon l'une quelconque des revendications 10 ou 11, **caractérisée en ce que** le monomère hydrophobe à insaturation éthylénique est choisi parmi les acrylates ou les acrylamides de formule (1) suivante 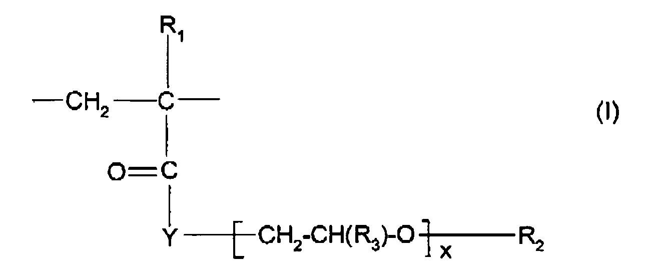 dans laquelle R₁ et R₃, identiques ou différents, désignent un atome d'hydrogène ou un radical alkyle linéaire ou ramifié en C₁-C₆ de préférence méthyle ; Y désigne O ou NH ; R₂ désigne un radical hydrocarboné hydrophobe comportant au moins de 10 à 18 atomes de carbone et encore plus particulièrement de 12 à 18 atomes de carbone ; x désigne un nombre de moles d'oxyde d'alkylène et varie de 0 à 100.

13. Composition de traitement selon la revendication 12, **caractérisée en ce que** le radical hydrophobe R₂ est choisi parmi 12 le radical cholestéryle ou un ester de cholestérol ; les groupes polycycliques aromatiques.

14. Composition de traitement selon l'une quelconque des revendications 12 ou 13, **caractérisée en ce que** le monomère de formule (1) comporte en plus au moins un motif oxyde d'alkylène (x ≥ 1).

15. Composition de traitement selon l'une quelconque des revendications 12 à 14, **caractérisée en ce que** le monomère de formule (I) comporte en plus au moins une chaîne polyoxyalkylénée.

16. Composition de traitement selon la revendication 15, **caractérisée en ce que** la chaîne polyoxyalkylénée est constituée de motifs oxyde d'éthylène et/ou de motifs oxyde de propylène.

17. Composition de traitement selon la revendication 16, **caractérisée en ce que** la chaîne polyoxyalkylénée est constituée uniquement de motifs oxyde d'éthylène.

18. Composition de traitement selon l'une quelconque des revendications 12 à 17, **caractérisée en ce que** le nombre de motifs oxyalkylénés varie de 3 à 100.

19. Composition de traitement selon la revendication **caractérisée en ce que** le nombre de motifs oxyalkylénés varie de 3 à 50.

20. Composition de traitement selon la revendication 19, **caractérisée en ce que** le nombre de motifs oxyalkylénés varie de 7 à 25.

21. Composition de traitement selon l'une quelconque des revendications 10 à 13, **caractérisée en ce que** le polymère sulfoné est choisi parmi :
- les copolymères non réticulés d'AMPS partiellement ou totalement neutralisé et de méthacrylate de n-dodécyle,
- les copolymères réticulés ou non réticulés d'AMPS partiellement ou totalement neutralisé et de n-dodécylméthacrylamide.

22. Composition de traitement selon l'une quelconque des revendications 10 à 20 **caractérisée en ce que** le polymère sulfoné est choisi parmi les copolymères constitués de motifs acide 2-acrylamido-2-méthylpropane-sulfonique (AMPS) de formule (II) suivante : 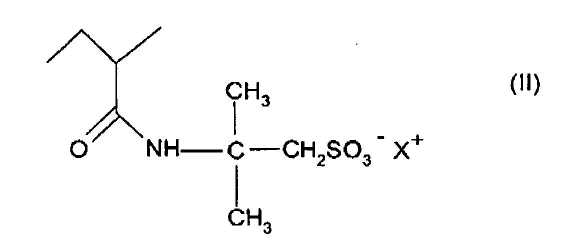 dans laquelle X⁺ est un proton, un cation de métal alcalin, un cation alcalino-tcrreux ou l'ion ammonium,
et de motifs de formule (III) suivante : 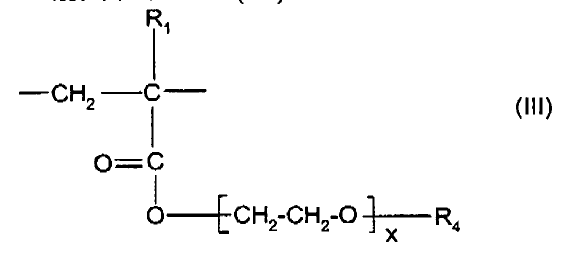 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₁₀-C₂₂.

23. Composition de traitement selon la revendication 22, **caractérisée en ce que** x = 25 , R₁ est méthyle et R₄ est n-dodécyle.

24. Composition de traitement selon la revendication 12 ou 22; **caractérisée en ce que** la proportion molaire en % des motifs de formule (I) ou des motifs de formule (III) dans les polymères varie de 50,1 à 99,9%.

25. Composition de traitement selon la revendication 12 ou 22, **caractérisée en ce que** la proportion molaire en % des motifs de formule (I) ou des motifs de formule (III) dans les polymères varie de 0,1 à 50 %.

26. Composition de traitement selon l'une quelconque des revendications précédentes, **caractérisée en ce que** les polymères sulfonés sont neutralisés partiellement ou totalement par une base minérale ou organique.

27. Composition de traitement selon l'une quelconque des revendications précédentes, **caractérisée en ce que** les polymères sulfonés ont un poids moléculaire moyen en nombre allant de 1000 à 20 000 000 g/mole.

28. Composition de traitement selon la revendication 27, **caractérisée en ce que** le poids moléculaire moyen en nombre varie de 20 000 à 5 000 000 g/mole.

29. Composition de traitement selon la revendication 28 **caractérisée en ce que** le poids moléculaire moyen en nombre varie de 100 000 à 1 500 000 g/mole.

30. Composition de traitement selon l'une quelconque des revendications précédentes, **caractérisée en ce qu'**une solution aqueuse à 1 % en poids desdits polymères présente à la température de 25°C une viscosité mesurée au viscosimètre Brookfield, aiguille 7, allant de 20 000 mPa.s à 100 000 mPa.s.

31. Composition de traitement selon l'une quelconque des revendications précédentes, **caractérisée en ce que** le ou les polymères sulfonés sont préparés par polymérisation radicalaire par précipitation dans le ter-butanol.

32. Composition de traitement selon l'une quelconque des revendications précédentes, **caractérisée en ce que** le ou les polymères sulfonés sont réticulés ou non-réticulés.

33. Composition de traitement selon la revendication 32. **caractérisée en ce que** le ou les polymères sulfonés sont réticulés.

34. Composition de traitement selon la revendication 33, **caractérisée en ce que** le ou les agents de réticulation sont choisis parmi les composés à polyinsaturation oléfinique.

35. Composition de traitement selon la revendication 34, **caractérisée en ce que** le ou les agents de réticulation sont choisis parmi le méthylène-bis-acrylamide, le méthacrylate d'allyle ou triméthylol propane triacrylate (TMPTA).

36. Composition de traitement selon l'une quelconque des revendications 33 à 35, **caractérisée en ce que** le taux de réticulation varie de préférence de 0,01 à 10 % en moles et plus particulièrement de 0,2 à 2 % en moles par rapport au polymère.

37. Composition de traitement selon l'une quelconque des revendications précédentes, **caractérisée en ce que** le polyuréthane fixant non associatif est présent en une quantité de 0,1 à 30 % en poids, de préférence de 0,2 à 15 % en poids, et encore plus préférentiellement de 0,5 à 10 % en poids par rapport au poids total de la composition.

38. Composition de traitement selon l'une quelconque des revendications précédentes, **caractérisée en ce que** le polymère sulfoné est présent en une quantité de 0,01 à 20 % en poids, de préférence de 0,1 à 10 % en poids et encore plus préférentiellement de 0,2 à 5 % en poids par rapport au poids total de la composition.

39. Composition de traitement selon l'une quelconque des revendications précédentes, **caractérisée en ce que** le milieu cosmétiquement acceptable comprend de l'eau et/ou un solvant cosmétiquement acceptable.

40. Composition de traitement selon la revendication 39, **caractérisée en ce que** le solvant cosmétiquement acceptable est choisi parmi les alcools inférieurs en C₁-C₄, les alkylènepolyols, les éthers de polyols et leurs mélanges.

41. Composition de traitement selon la revendication 39 ou 40, **caractérisée en ce que** le solvant cosmétiquement acceptable est choisi parmi l'éthanol, l'isopropanol, le tertio-butanol et le propylène-glycol.

42. Composition de traitement selon l'une quelconque des revendications précédentes, **caractérisée en ce qu'**elle comprend en outre un ou plusieurs additifs cosmétiques usuels choisis parmi les agents adhésifs, les agents réducteurs comme les thiols, les corps gras, les agents épaississants, les adoucissants, les agents nacrant, les agents anti-mousse, les filtres solaires, les agents antiperspirants, les agents acidifiants, les agents alcalinisants, les colorants, les pigments, les parfums, les conservateurs, les tensioactifs, les silicones volatiles ou non, les huiles végétales ou minérales, les protéines et les vitamines, et leurs mélanges.

43. Composition de traitement selon l'une quelconque des revendications précédentes, **caractérisée en ce qu'**elle se présente sous forme d'un gel, d'un liquide ou d'une mousse.

44. Composition de traitement selon l'une quelconque des revendications précédentes, **caractérisée en ce qu'**elle est conditionnée dans un tube, un vaporisateur ou un dispositif aérosol.

45. Procédé de traitement cosmétique capillaire, **caractérisé en ce que** l'on applique la composition selon l'une quelconque des revendications précédentes sur les cheveux, et que l'on rince ou après un éventuel temps de pose.

46. Utilisation de la composition selon l'une quelconque de revendications précédentes 1 à 44, comme produit de coiffage.

## Claims

1. Cosmetic hair treatment composition, **characterized in that** it comprises, in a cosmetically acceptable medium, at least one non-associative fixing polyurethane, the said polyurethane being a polycondensate comprising at least one polyurethane block capable of providing hairstyle hold and not comprising in its structure any terminal or pendent fatty chains comprising more than 10 carbon atoms, at least one optionally partially or totally neutralized associative sulfonated polymer, comprising at least one unit derived from an ethylenically unsaturated monomer containing a non-aromatic sulfonic group, the said associative sulfonated polymer bearing at least one hydrophilic , portion and at least one terminal or pendent fatty chain comprising at least 10 carbon atoms.

2. Treatment composition according to Claim 1, **characterized in that** the non-associative fixing polyurethane comprises a base repeating unit corresponding to the general formula (I):
-O-B-O-CO-NH-R-NH-CO- (I)
in which:
- B is a divalent C₁ to C₃₀ hydrocarbon-based group, this group being optionally substituted with a group comprising one or more carboxylic acid functions and/or one or more sulfonic acid functions, the said carboxylic and/or sulfonic acid functions being in free form or a form partially or totally neutralized with a mineral or organic base, and
- R is a divalent group chosen from alkylene groups of aromatic, C₁ to C₂₀ aliphatic or C₁ to C₂₀ cycloaliphatic type, these groups being substituted or unsubstituted.

3. Treatment composition according to Claim 1, **characterized in that** the non-associative fixing polyurethane comprises a base repeating unit corresponding to the general formula (II):
-O-P-O-CO-NH-R-NH-CO- (II)
in which:
- P is a polysiloxane block, and
- R is a divalent group chosen from alkylene groups of aromatic, C₁ to C₂₀ aliphatic or C₁ to C₂₀ cycloaliphatic type, these groups being substituted or unsubstituted.

4. Treatment composition according to Claim 1 or 2, **characterized in that** the non-associative fixing polyurethane is a dimethylolpropionic acid/isophorone diisocyanate/neopentyl glycol/polyester diol copolymer.

5. Treatment composition according to Claim 1 or 3, **characterized in that** the non-associative fixing polyurethane is a dimethylolpropionic acid/isophorone diisocyanate/neopentyl glycol/polyester diol/silicone diamine copolymer.

6. Treatment composition according to any one of the preceding claims, **characterized in that** the ethylenically unsaturated monomer containing a non-aromatic sulfonic group is chosen from vinylsulfonic acid, (meth)acrylamido (C₁-C₂₂)alkylsulfonic acids and N-(C₁-C₂₂) alkyl (meth) acrylamido (C₁-C₂₂) alkylsulfonic acids, and partially or totally neutralized forms thereof.

7. Treatment composition according to Claim 6, **characterized in that** the ethylenically unsaturated monomer containing a non-aromatic sulfonic group is chosen from acrylamidomethanesulfonic acid, acrylamido-ethanesulfonic acid, acrylamidopropanesulfonic acid, 2-acrylamido-2-methylpropanesulfonic acid, methacryl-amido-2-methylpropanesulfonic acid, 2-acrylamido-n-butane-sulfonic acid, 2-acrylamido-2,4,4-trimethylpentanesulfonic acid, 2-acrylamidododecylsulfonic acid and 2-acrylamido-2,6-dimethyl-3-heptanesulfonic acid, and also partially or totally neutralized forms thereof.

8. Treatment composition according to either of Claims 6 and 7, **characterized in that** the ethylenically unsaturated monomer containing a non-aromatic sulfonic group is 2-acrylamido-2-methylpropanesulfonic acid (AMPS), and also partially or totally neutralized forms thereof.

9. Treatment composition according to Claim 8, **characterized in that** the sulfonated polymer(s) also contain at least one unit derived from an ethylenically unsaturated monomer not comprising any fatty chains, chosen from (meth)acrylic acids and β-substituted alkyl derivatives thereof, and esters thereof obtained with monoalcohols or mono- or polyalkylene glycols, or alternatively from (meth)acrylamides, vinylpyrrolidone, maleic anhydride, itaconic acid and maleic acid, or mixtures of these compounds.

10. Treatment composition according to any one of Claims 1 to 8, **characterized in that** the sulfonated polymer(s) are chosen from amphiphilic copolymers of AMPS and of at least one ethylenically unsaturated hydrophobic monomer comprising at least one hydrophobic portion containing from 10 to 18 carbon atoms.

11. Treatment composition according to Claim 10, **characterized in that** the hydrophobic portion comprises from 12 to 18 carbon atoms.

12. Treatment composition according to either of Claims 10 and 11, **characterized in that** the ethylenically unsaturated hydrophobic monomer is chosen from the acrylates or acrylamides of formula (I) below: 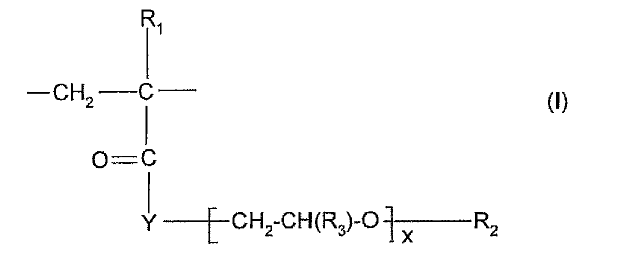 in which R₁ and R₃, which may be identical or different, denote a hydrogen atom or a linear or branched C₁-C₆ alkyl radical, preferably methyl; Y denotes O or NH; R₂ denotes a hydrophobic hydrocarbon-based radical comprising at least from 10 to 18 carbon atoms and even more particularly from 12 to 18 carbon atoms; x denotes a number of moles of alkylene oxide and ranges from 0 to 100.

13. Treatment composition according to Claim 12, **characterized in that** the hydrophobic radical R₂ is chosen from the cholesteryl radical or a cholesterol ester; aromatic polycyclic groups.

14. Treatment composition according to either of Claims 12 and 13, **characterized in that** the monomer of formula (I) also comprises at least one alkylene oxide unit (x ≥ 1).

15. Treatment composition according to any one of Claims 12 to 14, **characterized in that** the monomer of formula (I) also comprises at least one polyoxyalkylene chain.

16. Treatment composition according to Claim 15, **characterized in that** the polyoxyalkylene chain consists of ethylene oxide units and/or propylene oxide units.

17. Treatment composition according to Claim 16, **characterized in that** the polyoxyalkylene chain consists solely of ethylene oxide units.

18. Treatment composition according to any one of Claims 12 to 17, **characterized in that** the number of oxyalkylene units ranges from 3 to 100.

19. Treatment composition according to Claim 18, **characterized in that** the number of oxyalkylene units ranges from 3 to 50.

20. Treatment composition according to Claim 19, **characterized in that** the number of oxyalkylene units ranges from 7 to 25.

21. Treatment composition according to any one of Claims 10 to 13, **characterized in that** the sulfonated polymer is chosen from:
- non-crosslinked copolymers of partially or totally neutralized AMPS and of n-dodecyl methacrylate,
- crosslinked or non-crosslinked copolymers of partially or totally neutralized AMPS and of n-dodecylmethacrylamide.

22. Treatment composition according to any one of Claims 10 to 20, **characterized in that** the sulfonated polymer is chosen from copolymers consisting of 2-acrylamido-2-methylpropanesulfonic acid (AMPS) units of formula (II) below: 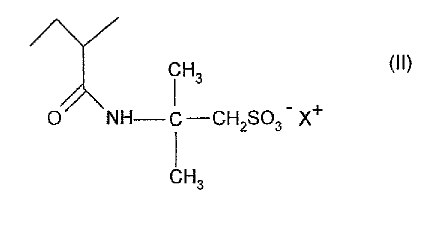 in which X⁺ is a proton, an alkali metal cation, an alkaline-earth metal cation or an ammonium ion,
and units of formula (III) below: 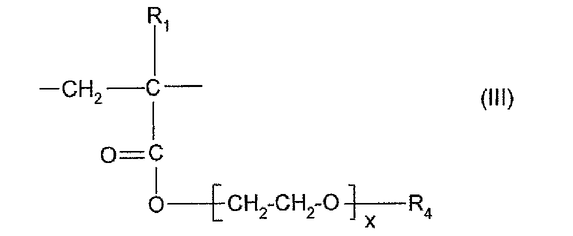 in which x denotes an integer ranging from 3 to 100, preferably from 5 to 80 and more preferentially from 7 to 25; R₁ has the same meaning as that indicated above in formula (I) and R₄ denotes a linear or branched C₁₀-C₂₂ alkyl.

23. Treatment composition according to Claim 22, **characterized in that** x = 25, R₁ is methyl and R₄ is n-dodecyl.

24. Treatment composition according to Claim 12 or 22, **characterized in that** the percentage mole proportion of units of formula (I) or of units of formula (III) in the polymers ranges from 50.1% to 99.9%.

25. Treatment composition according to Claim 12 or 22, **characterized in that** the percentage mole proportion of units of formula (I) or of units of formula (III) in the polymers ranges from 0.1% to 50%.

26. Treatment composition according to any one of the preceding claims, **characterized in that** the sulfonated polymers are partially or totally neutralized with a mineral or organic base.

27. Treatment composition according to any one of the preceding claims, **characterized in that** the sulfonated polymers have a number-average molecular weight ranging from 1000 to 20 000 000 g/mol.

28. Treatment composition according to Claim 27, **characterized in that** the number-average molecular weight ranges from 20 000 to 5 000 000 g/mol.

29. Treatment composition according to Claim 28, **characterized in that** the number-average molecular weight ranges from 100 000 to 1 500 000 g/mol.

30. Treatment composition according to any one of the preceding claims, **characterized in that** an aqueous solution at 1% by weight of the said polymers has at a temperature of 25°C a viscosity, measured using a Brookfield viscometer with a No. 7 needle, ranging from 20 000 mPa.s to 100 000 mPa.s.

31. Treatment composition according to any one of the preceding claims, **characterized in that** the sulfonated polymer(s) are prepared by radical polymerization by precipitation in tert-butanol.

32. Treatment composition according to any one of the preceding claims, **characterized in that** the sulfonated polymer(s) are crosslinked or non-crosslinked.

33. Treatment composition according to Claim 32, **characterized in that** the sulfonated polymer(s) are crosslinked.

34. Treatment composition according to Claim 33, **characterized in that** the crosslinking agent(s) are chosen from polyolefinically unsaturated compounds.

35. Treatment composition according to Claim 34, **characterized in that** the crosslinking agent(s) are chosen from methylenebisacrylamide, allyl methacrylate and trimethylolpropane triacrylate (TMPTA).

36. Treatment composition according to any one of Claims 33 to 35, **characterized in that** the degree of crosslinking preferably ranges from 0.01 mol% to 10 mol% and more particularly from 0.2 mol% to 2 mol% relative to the polymer.

37. Treatment composition according to any one of the preceding claims, **characterized in that** the non-associative fixing polyurethane is present in an amount of from 0.1% to 30% by weight, preferably from 0.2% to 15% by weight and even more preferentially from 0.5% to 10% by weight relative to the total weight of the composition.

38. Treatment composition according to any one of the preceding claims, **characterized in that** the sulfonated polymer is present in an amount ranging from 0.01% to 20% by weight, preferably from 0.1% to 10% by weight and even more preferentially from 0.2% to 5% by weight relative to the total weight of the composition.

39. Treatment composition according to any one of the preceding claims, **characterized in that** the cosmetically acceptable medium comprises water and/or a cosmetically acceptable solvent.

40. Treatment composition according to Claim 39, **characterized in that** the cosmetically acceptable solvent is chosen from C₁-C₄ lower alcohols, alkylene polyols and polyol ethers, and mixtures thereof.

41. Treatment composition according to Claim 39 or 40, **characterized in that** the cosmetically acceptable solvent is chosen from ethanol, isopropanol, tert-butanol and propylene glycol.

42. Treatment composition according to any one of the preceding claims, **characterized in that** it also comprises one or more common cosmetic additives chosen from adhesives, reducing agents such as thiols, fatty substances, thickeners, softeners, nacres, antifoams, sunscreens, antiperspirants, acidifying agents, basifying agents, dyes, pigments, fragrances, preserving agents, surfactants, volatile or nonvolatile silicones, plant or mineral oils, proteins and vitamins, and mixtures thereof.

43. Treatment composition according to any one of the preceding claims, **characterized in that** it is in the form of a gel, a liquid or a foam.

44. Treatment composition according to any one of the preceding claims, **characterized in that** it is conditioned in a tube, a vaporizer or an aerosol device.

45. Cosmetic hair treatment process, **characterized in that** the composition according to any one of the preceding claims is applied to the hair, and is rinsed out after an optional leave-on time.

46. Use of the composition according to any one of the preceding Claims 1 to 44, as a hairstyling product.

## Patentansprüche

1. Kosmetische Haarbehandlungszusammensetzung, **dadurch gekennzeichnet, dass** sie in einem kosmetisch annehmbaren Medium Folgendes umfasst: mindestens ein fixierendes, nicht assoziatives Polyurethan, wobei das Polyurethan ein Polykondensat ist, das mindestens eine Polyurethan-Abfolge enthält, die die Erhaltung der Frisur herbeiführen kann und in seiner Struktur keine endständige oder anhängende Fettsäurekette mit mehr als 10 Kohlenstoffatomen umfasst, und mindestens ein assoziatives, teilweise oder vollständig neutralisiertes oder nicht neutralisiertes sulfoniertes Polymer, das mindestens ein Motiv umfasst, das von einem ethylenisch ungesättigten Monomer mit nicht-aromatischer Sulfonsäuregruppe stammt, wobei das assoziative sulfonierte Polymer mindestens einen hydrophilen Teil und mindestens eine endständige oder anhängende Fettsäurekette mit mindestens 10 Kohlenstoffatomen umfasst.

2. Behandlungszusammensetzung nach Anspruch 1, **dadurch gekennzeichnet, dass** das nicht assoziative, fixierende Polyurethan ein wiederholtes Grundmotiv umfasst, das der allgemeinen Formel (I) entspricht:
-O-B-O-CO-NH-R-NH-CO- (I)
worin:
- B eine zweiwertige C₁- bis C₃₀-Kohlenwasserstoffgruppe ist, wobei diese Gruppe unsubstituiert oder mit einer Gruppe substituiert ist, die eine oder mehrere Carbonsäurefunktionen und/oder eine oder mehrere Sulfonsäurefunktionen trägt, wobei diese Carbonsäure- und/oder Sulfonsäurefunktionen in freier Form vorliegen oder auch teilweise oder vollständig durch eine anorganische oder organische Base neutralisiert sind, und
- R eine zweiwertige Gruppe ist, die aus den Alkylengruppen vom aromatischen Typ, aliphatischen Typ mit 1 bis 20 Kohlenstoffatomen, cycloaliphatischen Typ mit 1 bis 20 Kohlenstoffatomen ausgewählt ist, wobei diese Gruppen substituiert oder unsubstituiert sind.

3. Behandlungszusammensetzung nach Anspruch 1, **dadurch gekennzeichnet, dass** das nicht assoziative, fixierende Polyurethan ein wiederholtes Grundmotiv umfasst, das der allgemeinen Formel (II) entspricht:
-O-P-O-CO-NH-R-NH-CO- (II)
worin:
- P ein Polysiloxan-Segment ist und
- R eine zweiwertige Gruppe ist, die aus den Alkylengruppen vom aromatischen Typ, aliphatischen Typ mit 1 bis 20 Kohlenstoffatomen, cycloaliphatischen Typ mit 1 bis 20 Kohlenstoffatomen ausgewählt ist, wobei diese Gruppen substituiert oder unsubstituiert sind.

4. Behandlungszusammensetzung nach Anspruch 1 oder 2, **dadurch gekennzeichnet, dass** das fixierende, nicht assoziative Polyurethan ein Dimethylolpropionsäure/Isophorondiisocyanat/Neopentylglykol/Polyesterdiole-Copolymer ist.

5. Behandlungszusammensetzung nach Anspruch 1 oder 3, **dadurch gekennzeichnet, dass** das fixierende, nicht assoziative Polyurethan ein silikonisiertes Dimethylolpropionsäure/Isophorondiisocyanat/Neopentylglykol/Polyesterdiole/Diamin-Copolymer ist.

6. Behandlungszusammensetzung nach einem der vorhergehenden Ansprüche, **dadurch gekennzeichnet, dass** das ethylenisch ungesättigte Monomer mit nicht-aromatischer Sulfonsäuregruppe aus Vinylsulfonsäure, (Meth) Acrylamido (C₁-C₂₂) alkylsulfonsäuren, N- (C₁-C₂₂) Alkyl (meth) acrylamido (C₁-C₂₂) alkylsulfonsäuren sowie deren teilweise oder vollständig neutralisierten Formen ausgewählt wird.

7. Behandlungszusammensetzung nach Anspruch 6, **dadurch gekennzeichnet, dass** das ethylenisch ungesättigte Monomer mit nicht-aromatischer Sulfonsäuregruppe aus Acrylamidomethansulfonsäure, Acrylamidoethansulfonsäure, Acrylamidopropansulfonsäure, 2-Acrylamido-2-methylpropansulfonsäure, Methacrylamido-2-methylpropansulfonsäure, 2-Acrylamido-n-butansulfonsäure, 2-Acrylamido-2,4,4-trimethylpentansulfonsäure, 2-Methacrylamidododecylsulfonsäure, 2-Acrylamido-2,6-dimethyl-3-heptansulfonsäure sowie deren teilweise oder vollständig neutralisierten Formen ausgewählt wird.

8. Behandlungszusammensetzung nach einem der Ansprüche 6 oder 7, **dadurch gekennzeichnet, dass** das ethylenisch ungesättigte Monomer mit nicht-aromatischer Sulfonsäuregruppe 2-Acrylamido-2-methylpropansulfonsäure (AMPS) sowie dessen teilweise oder vollständig neutralisierte Formen ist.

9. Behandlungszusammensetzung nach Anspruch 8, **dadurch gekennzeichnet, dass** das oder die sulfonierte(n) Polymer(e) außerdem mindestens ein Motiv enthält/enthalten, das von einem Monomer mit ethylenischer Ungesättigheit stammt, das keine Fettsäurekette enthält, ausgewählt aus (Meth)Acrylsäuren und ihren an ß alkylsubstituierten Derivaten und ihren Estern, die mit Monoalkoholen oder Mono- oder Polyalkylenglykolen erhalten werden, oder auch aus (Meth)Acrylamiden, Vinylpyrrolidon, Maleinsäureanhydrid, Itaconsäure oder Maleinsäure oder den Gemischen dieser Verbindungen.

10. Behandlungszusammensetzung nach einem der Ansprüche 1 bis 8, **dadurch gekennzeichnet, dass** das oder die sulfonierte(n) Polymer(e) aus den amphiphilen Copolymeren von AMPS und mindestens einem hydrophoben Monomer mit ethylenischer Ungesättigtheit, das mindestens einen hydrophoben Anteil mit 10 bis 18 Kohlenstoffatomen enthält, ausgewählt wird/werden.

11. Behandlungszusammensetzung nach Anspruch 10, **dadurch gekennzeichnet, dass** der hydrophobe Anteil 12 bis 18 Kohlenstoffatome enthält.

12. Behandlungszusammensetzung nach einem der Ansprüche 10 oder 11, **dadurch gekennzeichnet, dass** das hydrophobe Monomer mit ethylenischer Ungesättigtheit ausgewählt wird aus den Acrylaten oder Acrylamiden der folgenden Formel (I) 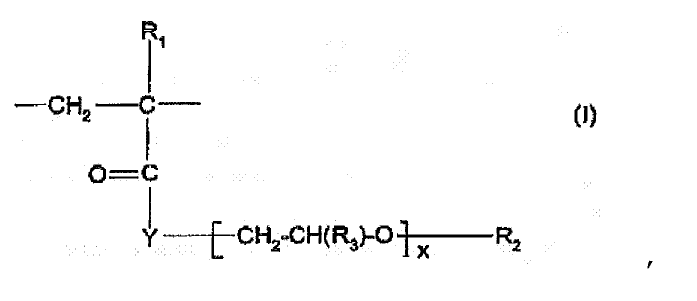 worin R₁ und R₃, die gleich oder verschieden sind, für ein Wasserstoffatom oder einen geraden oder verzweigten C₁-C₆-Alkylrest, vorzugsweise Methyl, stehen; Y für O oder NH steht; R₂ für einen hydrophoben Kohlenwasserstoffrest steht, der mindestens 10 bis 18 Kohlenstoffatome und noch stärker bevorzugt 12 bis 18 Kohlenstoffatome enthält; x für eine Anzahl der Mole an Alkylenoxid steht und von 0 bis 100 variiert.

13. Behandlungszusammensetzung nach Anspruch 12, **dadurch gekennzeichnet, dass** der hydrophobe Rest R₂ aus einem Cholesterylrest oder einem Ester von Cholesterin, aromatischen polycyclischen Gruppen ausgewählt wird.

14. Behandlungszusammensetzung nach einem der Ansprüche 12 oder 13, **dadurch gekennzeichnet, dass** das Monomer der Formel (I) außerdem mindestens ein Alkylenoxid-Motiv enthält (x ≥ 1).

15. Behandlungszusammensetzung nach einem der Ansprüche 12 bis 14, **dadurch gekennzeichnet, dass** das Monomer der Formel (I) mindestens eine polyoxyalkylenierte Kette enthält.

16. Behandlungszusammensetzung nach Anspruch 15, **dadurch gekennzeichnet, dass** die polyoxyalkylenierte Kette aus Ethylenoxid-Motiven und/oder Propylenoxid-Motiven besteht.

17. Behandlungszusammensetzung nach Anspruch 16, **dadurch gekennzeichnet, dass** die polyoxyalkylenierte Kette nur aus Ethylenoxid-Motiven besteht.

18. Behandlungszusammensetzung nach einem der Ansprüche 12 bis 17, **dadurch gekennzeichnet, dass** die Anzahl der Oxyalkylen-Motive von 3 bis 100 variiert.

19. Behandlungszusammensetzung nach Anspruch 18, **dadurch gekennzeichnet, dass** die Anzahl der Oxyalkylen-Motive von 3 bis 50 variiert.

20. Behandlungszusammensetzung nach Anspruch 19, **dadurch gekennzeichnet, dass** die Anzahl der Oxyalkylen-Motive von 7 bis 25 variiert.

21. Behandlungszusammensetzung nach einem der Ansprüche 10 bis 13, **dadurch gekennzeichnet, dass** das sulfonierte Polymer ausgewählt wird aus:
- den nicht vernetzten Copolymeren aus teilweise oder vollständig neutralisiertem AMPS und n-Dodecylmethacrylat,
- den vernetzten oder nicht vernetzten Copolymeren aus teilweise oder vollständig neutralisiertem AMPS und n-Dodecylmethacrylamid.

22. Behandlungszusammensetzung nach einem der Ansprüche 10 bis 20, **dadurch gekennzeichnet, dass** das sulfonierte Polymer aus Copolymeren ausgewählt wird, bestehend aus 2-Acrylamido-2-methylpropansulfonsäure-(AMPS-) Motiven der folgenden Formel (II): 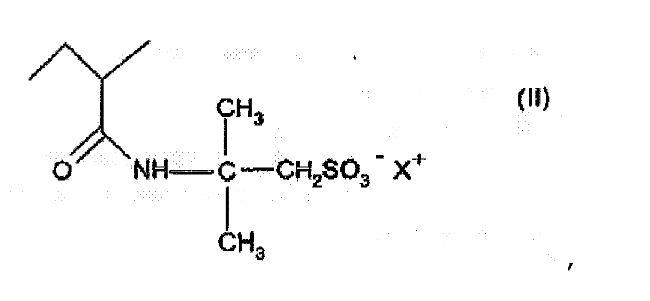 worin X⁺ ein Proton, ein Alkalimetallkation, ein Erdalkalimetallkation oder das Ammoniumion ist,
und Motiven der folgenden Formel (III): 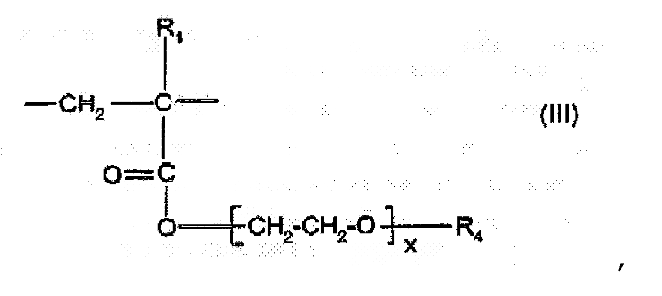 worin x für eine ganze Zahl von 3 bis 100, vorzugsweise von 5 bis 80 und stärker bevorzugt von 7 bis 25 steht; R₁ die gleiche Bedeutung hat, wie vorstehend bei Formel (I) angegeben, und R₄ für ein gerades oder verzweigtes C₁₀-C₂₂-Alkyl steht.

23. Behandlungszusammensetzung nach Anspruch 22, **dadurch gekennzeichnet, dass** x = 25 , R₁ Methyl ist und R₄ n-Dodecyl ist.

24. Behandlungszusammensetzung nach Anspruch 12 oder 22, **dadurch gekennzeichnet, dass** der molare Anteil in % der Motive der Formel (I) oder der Motive der Formel (III) in den Polymeren von 50,1 bis 99,9% variiert.

25. Behandlungszusammensetzung nach Anspruch 12 oder 22, **dadurch gekennzeichnet, dass** der molare Anteil in % der Motive der Formel (I) oder der Motive der Formel (III) in den Polymeren von 0,1 bis 50 % variiert.

26. Behandlungszusammensetzung nach einem der vorhergehenden Ansprüche, **dadurch gekennzeichnet, dass** die sulfonierten Polymere teilweise oder vollständig mit einer anorganischen oder organischen Base neutralisiert sind.

27. Behandlungszusammensetzung nach einem der vorhergehenden Ansprüche, **dadurch gekennzeichnet, dass** die sulfonierten Polymere eine zahlengemittelte Molmasse von 1000 bis 20000000 g/mol besitzen.

28. Behandlungszusammensetzung nach Anspruch 27, **dadurch gekennzeichnet, dass** die zahlengemittelte Molmasse von 20000 bis 5000000 g/mol variiert.

29. Behandlungszusammensetzung nach Anspruch 28, **dadurch gekennzeichnet, dass** die zahlengemittelte Molmasse von 100000 bis 1500000 g/mol variiert.

30. Behandlungszusammensetzung nach einem der vorhergehenden Ansprüche, **dadurch gekennzeichnet, dass** eine wässrige 1 Gew.-%ige Lösung der Polymere bei einer Temperatur von 25°C eine im Brookfield-Viskosimeter, Spindel 7, gemessene Viskosität von 20000 mPa.s bis 100000 mPa.s aufweist.

31. Behandlungszusammensetzung nach einem der vorhergehenden Ansprüche, **dadurch gekennzeichnet, dass** das oder die sulfonierte(n) Polymer(e) durch radikalische Polymerisation durch Fällung aus tert.-Butanol hergestellt wird/werden.

32. Behandlungszusammensetzung nach einem der vorhergehenden Ansprüche, **dadurch gekennzeichnet, dass** das oder die sulfonierte(n) Polymer(e) vernetzt oder nicht vernetzt ist/sind.

33. Behandlungszusammensetzung nach Anspruch 32, **dadurch gekennzeichnet, dass** das oder die sulfonierte(n) Polymer(e) vernetzt ist/sind.

34. Behandlungszusammensetzung nach Anspruch 33, **dadurch gekennzeichnet, dass** das oder die Vernetzungsmittel aus mehrfach olefinisch ungesättigten Verbindungen ausgewählt wird/werden.

35. Behandlungszusammensetzung nach Anspruch 34, **dadurch gekennzeichnet, dass** das oder die Vernetzungsmittel aus Methylenbisacrylamid, Allylmethacrylat oder Trimethylolpropantriacrylat (TMPTA) ausgewählt wird/werden.

36. Behandlungszusammensetzung nach einem der Ansprüche 33 bis 35, **dadurch gekennzeichnet, dass** der Vernetzungsgrad vorzugsweise von 0,01 bis 10 Mol-% und stärker bevorzugt von 0,2 bis 2 Mol-%, bezogen auf das Polymer, variiert.

37. Behandlungszusammensetzung nach einem der vorhergehenden Ansprüche, **dadurch gekennzeichnet, dass** das fixierende, nicht assoziative Polyurethan in einer Menge von 0,1 bis 30 Gew.-%, vorzugsweise von 0,2 bis 15 Ges.-% und noch stärker bevorzugt von 0,5 bis 10 Gew.-%, bezogen auf das Gesamtgewicht der Zusammensetzung, vorliegt.

38. Behandlungszusammensetzung nach einem der vorhergehenden Ansprüche, **dadurch gekennzeichnet, dass** das sulfonierte Polymer in einer Menge von 0,01 bis 20 Ges.-%, vorzugsweise von 0,1 bis 10 Gew.-% und noch stärker bevorzugt von 0,2 bis 5 Gew.-%, bezogen auf das Gesamtgewicht der Zusammensetzung, vorliegt.

39. Behandlungszusammensetzung nach einem der vorhergehenden Ansprüche, **dadurch gekennzeichnet, dass** das kosmetisch annehmbare Medium Wasser und/oder ein kosmetisch annehmbares Lösungsmittel umfasst.

40. Behandlungszusammensetzung nach Anspruch 39, **dadurch gekennzeichnet, dass** das kosmetisch annehmbare Lösungsmittel aus Niederalkoholen mit 1 bis 4 Kohlenstoffatomen, Alkylenpolyolen, Ethern von Polyolen und ihren Gemischen ausgewählt wird.

41. Behandlungszusammensetzung nach Anspruch 39 oder 40, **dadurch gekennzeichnet, dass** das kosmetisch annehmbare Lösungsmittel aus Ethanol, Isopropanol, tert.-Butanol und Propylenglykol ausgewählt wird.

42. Behandlungszusammensetzung nach einem der vorhergehenden Ansprüche, **dadurch gekennzeichnet, dass** sie außerdem einen oder mehrere übliche kosmetische Hilfsstoffe umfasst, die aus Klebemitteln, Reduktionsmitteln, wie Thiolen, Fettkörpern, Verdickungsmitteln, Weichmachern, Perlmuttglanz verleihenden Mitteln, Antischaummitteln, Sonnenfiltern, Antiperspirantien, Säuerungsmitteln, Alkalinisierungsmitteln, Farbstoffen, Pigmenten, Parfüms, Konservierungsmitteln, oberflächenaktiven Mitteln, flüchtigen oder nicht flüchtigen Silikonen, Pflanzen- oder Mineralölen, Proteinen und Vitaminen und deren Gemischen ausgewählt werden.

43. Behandlungszusammensetzung nach einem der vorhergehenden Ansprüche, **dadurch gekennzeichnet, dass** sie in Form eines Gels, einer Flüssigkeit oder eines Schaums vorliegt.

44. Behandlungszusammensetzung nach einem der vorhergehenden Ansprüche, **dadurch gekennzeichnet, dass** sie in einer Tube, einer Spritzflasche oder einer Sprühvorrichtung abgepackt ist.

45. Verfahren zur kosmetischen Haarbehandlung, **dadurch gekennzeichnet, dass** man die Zusammensetzung nach einem der vorhergehenden Ansprüche auf die Haare anwendet und dass man sie ausspült oder gegebenenfalls nach einer Einwirkzeit.

46. Verwendung der Zusammensetzung nach einem der vorhergehenden Ansprüche 1 bis 44 als Frisierprodukt.
